Europäisches Patentamt

European Patent Office

Office européen des brevets

(11) Veröffentlichungsnummer:

# 0 033 960
## A2

(12) ## EUROPÄISCHE PATENTANMELDUNG

(21) Anmeldenummer: 81100863.0

(51) Int. Cl.³: **G 01 N 33/54**

(22) Anmeldetag: **06.02.81**

(30) Priorität: **08.02.80 US 120017**

(43) Veröffentlichungstag der Anmeldung: **19.08.81**
Patentblatt 81/33

(84) Benannte Vertragsstaaten: **AT BE CH DE FR GB IT LI NL SE**

(71) Anmelder: **F. HOFFMANN-LA ROCHE & CO. Aktiengesellschaft, CH-4002 Basel (CH)**

(72) Erfinder: **Hansen, Hans John, 45 Maple Street, Allendale, N.J. (US)**
Erfinder: **Myl, Alfred David, 64 Stone Avenue, Elmwood Park, N.J. (US)**
Erfinder: **Vandevoorde, Jacques Pierre, 9 Nutting Place, West Caldwell, N.J. (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(54) Verfahren zur Vorbehandlung von Proben, welche für die Bestimmung von carcinoembryonischem Antigen verwendet werden.

(57) Es wird ein vereinfachtes Verfahren zur Vorbehandlung von Proben beschrieben, welche für die Bestimmung von carcinoembryonischem Antigen verwendet werden. Das verbesserte Verfahren besteht im wesentlichen darin, dass der Einfluss der möglicherweise interferierenden Substanzen dadurch aufgehoben wird, dass die Probe mit Wasser verdünnt wird und auf eine Temperatur erwärmt wird, welche unterhalb derjenigen liegt, bei welcher bei der Ionenstärke und dem pH der Probe die vorhandenen Proteine koagulieren würden.

F.Hoffmann-La Roche & Co.Aktiengesellschaft, Basel/Schweiz

RAN 4060/109

## Verfahren zur Vorbehandlung von Proben, welche für die Bestimmung von carcinoembryonischem Antigen verwendet werden.

Die Bestimmung von carcinoembryonischem Antigen (im nachfolgenden CEA genannt) ist der Fachwelt bestens bekannt. Der Fachwelt ist ebenfalls bekannt, dass gewisse, nicht-spezifisch interferierende Substanzen, welche in der zu testenden Probe vorhanden sind, weitgehend entfernt oder neutralisiert werden müssen, damit eine exakte und empfindliche Bestimmung durchgeführt werden kann.

Es sind eine Reihe von Verfahren bekannt, nach denen möglicherweise interferierende Substanzen, die in der Probe der biologischen Flüssigkeit, z.B. in der Serum- oder Plasmaprobe, vorhanden sind, vor Bestimmung des CEA entfernt oder neutralisiert werden können. Es bedeutet klarerweise hinsichtlich Zeit, Kosten und Einfachheit der Durchführung des Tests einen Vorteil, die zur Entfernung solcher interferierenden Substanzen erforderlichen Handhabungen in einem gegebenen Test zu vereinfachen.

Bei der Bestimmung von CEA gemäss Freedman et al. (US-Patent Nr. 3,663,684) wird die Blutprobe zuerst mit einem Glycoprotein-Lösungsmittel behandelt, in dem CEA löslich ist, worauf die resultierende Lösung geklärt wird.

Klt/12.1.1981

Beispiele solcher Lösungsmittel umfassen Perchlorsäure, Trichloressigsäure, Phosphorwolframsäure und dergleichen. Die Behandlung der Blutprobe mit dem Glycoprotein-Lösungsmittel erfolgt zum Zweck die fällbaren Normalproteine und die interferierenden Antigenmaterialien zu entfernen. Durch nachfolgende Zentrifugation der Probe werden die ausgefällten interferierenden Proteine entfernt.

Erst neulich haben Hirai, Cancer Research 37, 2267-2274, Juli 1977, und Kim et al., Clinical Chemistry 25, No. 5, 773-776, 1979, eine Probenvorbehandlungsmethode beschrieben, bei der die Probe anstelle der Extraktion mit einem Glycoprotein-Lösungsmittel, wie Perchlorsäure, einer Hitzebehandlung unterzogen wird. Die letzt erwähnte Publikation beschreibt eine Probenbehandlung, bei der die interferierenden Substanzen durch Puffern der Probe auf einen pH von 4,8 - 5 mit Acetatpuffer und Erwärmen der Probe während ungefähr 10-20 Minuten auf ungefähr 70-80°C entfernt werden. Diese Hitzebehandlung in Gegenwart eines Puffers mit hoher Ionenstärke und bei saurem pH bewirkt die Hitzedenaturation der interferierenden Proteine, welche koagulieren. Es ist deshalb erforderlich, dass die Probe zur Entfernung des koagulierten Materials zentrifugiert wird. Dieses Verfahren ist insofern unvorteilhaft, indem in der Probe vorhandenes CEA im koagulierten Material eingebettet werden kann und dadurch von der zu testenden Probe entfernt wird, wodurch die Genauigkeit der nachfolgenden Bestimmung beeinträchtigt wird.

Gemäss der vorliegenden Erfindung wurde ein Verfahren zur Vorbehandlung einer Humanserum- oder -plasma-Probe für die anschliessende Bestimmung von CEA gefunden, welches schneller, einfacher und weniger aufwendig ist als die bereits bekannten präparativen Methoden und welche den zusätzlichen Vorteil hat, dass die Probe nicht zentrifugiert werden muss.

- 3 - 　　　　0033960

Gemäss der vorliegenden Erfindung wird eine Methode zur Vorbehandlung von Proben von Human-Serum oder -Plasma zur Bestimmung von carcinoembryonischem Antigen (CEA) beschrieben, welche rasch, bequem und weniger aufwendig ist als die Methoden, welche bisher verwendet wurden. Die Methode gemäss vorliegender Erfindung besteht darin, dass man die Probe mit Wasser verdünnt und auf eine Temperatur erwärmt, welche unterhalb derjenigen liegt, bei welcher bei der Ionenstärke und dem pH der Probe die vorhandenen Proteine koagulieren würden, d.h. auf eine Temperatur von ungefähr 85 bis ungefähr 105°C, vorzugsweise ungefähr 95°C. Die Erwärmung der Probe erfolgt während einer relativ kurzen Zeit.

Beim Hitzeschritt gemäss vorliegender Erfindung wird die Serum- oder Plasma-Probe eines Patienten, welche zu testen ist, während ungefähr 3 bis ungefähr 30 Minuten, vorzugsweise während ungefähr 5 bis 10 Minuten, erwärmt. Die Dauer, während der die Probe bei der oben erwähnten Temperatur gehalten wird, ist nicht besonders kritisch. Es ist lediglich notwendig, dass die Probe auf die oben erwähnte Temperatur erwärmt wird und vorzugsweise während einer kurzen Zeit, z.B. während 5 bis 10 Minuten, bei dieser Temperatur gehalten wird. Aus diesem Grund ist die Dauer des Erwärmens lediglich zur Illustration angegeben; es ist lediglich notwendig, dass die Probe zur gewünschten Temperatur erwärmt und während einer kurzen Zeit bei dieser Temperatur gehalten wird, um sicherzustellen, dass die gesamte Probe die gewünschte Temperatur erreicht hat. Gemäss vorliegender Erfindung muss - wie früher erwähnt - die Probe auf eine Temperatur erwärmt werden, welche unterhalb derjenigen liegt, bei welcher bei niedriger Ionenstärke und neutralem bis leicht alkalischem pH keine Proteine koagulieren.

Die Verdünnung der Testprobe mit Wasser ist notwendig, um die Ionenstärke der Probe niedrig, d.h. höchstens bei einer Ionenstärke von einem Achtel der normalen physiologischen Ionenstärke zu halten. Obwohl Leitungswasser beim Verdünnungsschritt verwendet werden kann, wird destilliertes oder deionisiertes Wasser bevorzugt. Gewöhnlich wird eine Verdünnung von ungefähr 1:8 bis ungefähr 1:50 verwendet, wobei eine bevorzugte Verdünnung 1:16 ist. Die so verdünnte Probe hat eine niedrige Ionenstärke und einen neutralen oder leicht alkalischen pH, d.h. einen pH von ungefähr 7 bis ungefähr 9. Es wurde gefunden, dass es beim oben erwähnten Verdünnungsbereich üblicherweise nicht notwendig ist, die Probe mit Puffer enthaltendem Wasser zu verdünnen. Zur Optimalisierung der Empfindlichkeit der nachfolgenden Bestimmung kann es jedoch vorteilhaft sein, dem Wasser, das zur Verdünnung benutzt wird, einen Puffer zuzusetzen, um sicherzustellen, dass der pH bei einem leicht alkalischen Niveau gehalten wird. Sofern bei der Verdünnung der Probe ein Puffer verwendet wird, sind herkömmliche alkalische Puffer, wie z.B. ein Tris(hydroxymethyl)aminomethan-Puffer, geeignet. Es muss ein genügender Anteil an Puffer verwendet werden, um sicherzustellen, dass der pH der Probe leicht alkalisch ist, d.h. in einem pH-Bereich von ungefähr 7,5 bis ungefähr 9,0 liegt.

Zusätzlich zum erwähnten Vorteil, dass die Probe im Gegensatz zu den herkömmlichen Hitzebehandlungen nicht zentrifugiert werden muss, zeigt die erfindungsgemässe Vorbehandlungsmethode den weiteren Vorteil, dass die nachfolgende Bestimmung mit kleineren Mengen an Proben, d.h. ungefähr 100 µl, durchgeführt werden kann, welche geringer sind als diejenigen, welche bislang als praktisch angesehen wurden. So z.B. wird beim gegenwärtigen kommerziellen Radioimmunverfahren für CEA eine 0,5 ml-Probe verwendet. Die Verminderung des Probenvolumens zur Bestimmung von CEA im Vergleich mit den bekannten Bestimmungsmethoden führt zu keinem signifikanten Verlust in der Empfindlichkeit der Bestimmung. Die bedeutenden Einsparungen hinsichtlich Zeit und Kosten, welche durch die Verwendung der erfindungsgemässen Vorbehandlungsmethode erreicht werden, ermöglichen eine CEA-Bestimmung in relativ grossem Massstab, z.B. für Massenuntersuchungen. Eine vollständige CEA-Bestimmung unter Verwendung der erfindungsgemässen Vorbehandlungsmethode kann in ungefähr 4 Stunden durchgeführt werden. Dies bedeutet eine entscheidende Verbesserung gegenüber den kommerziellen CEA-Bestimmungsmethoden, bei denen eine Dialyse verwendet wird, welche über Nacht durchgeführt werden muss. Die CEA-Bestimmung unter Verwendung der Vorbehandlungsmethode gemäss vorliegender Erfindung bietet im weitern einen bedeutenden Fortschritt hinsichtlich des Empfindlichkeitsbereichs der Bestimmung gegenüber den bekannten Bestimmungsmethoden, wie dies später diskutiert werden soll.

Die Vorbehandlungsmethode gemäss vorliegender Erfindung umfasst im wesentlichen das Verdünnen der Plasma- oder Serum-Probe auf eine Verdünnung von ungefähr 1:8 zu ungefähr 1:50, vorzugsweise ungefähr 1:16, und Erwärmen der verdünnten Probe auf eine Temperatur, die unterhalb derjenigen liegt, bei der die in der Probe enthaltenen Proteine koagulieren würden, d.h. auf eine Temperatur von ungefähr 85 bis ungefähr 105°C, vorzugsweise 95°C. Das exakte Prinzip, nach welchem bei dieser Methode der Einfluss der interferierenden Proteine in der Probe, welche bislang physikalisch entfernt werden mussten, aufgehoben wird, ist nicht klar.

Die gemäss der vorliegenden Erfindung vorbehandelte Probe lässt man anschliessend auf Raumtemperatur abkühlen, worauf man eine geeignete Bestimmungsmethode für CEA durchführt. Die Wahl eines bestimmten Typs von Immunoassay für CEA, welcher dann verwendet werden soll, ist nicht kritisch. Im allgemeinen wird ein Radioimmun- oder ein Enzymimmun-Verfahren bevorzugt, wobei ein Radioimmun-Verfahren besonders bevorzugt wird.

Im allgemeinen umfasst eine Bestimmung von CEA die folgenden Schritte:

0033960

a) Vorbehandeln der Serum- oder Plasma-Probe eines Patienten, wie vorgehend beschrieben, um die möglicherweise interferierenden Materialien zu neutralisieren;

b) Zufügen eines Ueberschusses von CEA-Antikörpern zu den zu untersuchenden Proben und Inkubation während einer vorbestimmten Zeit;

c) Zufügen von markiertem CEA zu den Proben und Inkubation während einer vorgegebenen Zeit;

d) Zufügen eines Insolubilisierungsmittels zum Gemisch von b) und c) und Bildung einer Festphase enthaltend Antikörper-gebundenes CEA und eine flüssige Phase enthaltend ungebundenes CEA;

e) Trennen der festen und der flüssigen Phasen;

f) Bestimmen des Anteils von Markierungsmittel entweder in der festen oder der flüssigen Phase und

g) Bestimmen des Gehaltes an CEA in der Probe durch Vergleich mit einem Standard.

Bei der oben erwähnten Bestimmungsmethode wird der Begriff "neutralisieren" von möglicherweise interferierenden Materialien verwendet. Der Begriff "neutralisieren" bezieht sich sowohl auf die erfindungsgemässe Probenvorbehandlung wie die vorbekannten Methoden, bei denen diese interferierenden Materialien physikalisch abgetrennt werden, da ja der gewünschte Effekt in beiden Fällen derselbe ist. Im Prinzip bedeutet also der Begriff "neutralisieren", dass die interferierenden Materialien entfernt werden.

Wie früher erwähnt, kann das zur Markierung von CEA verwendete Markierungsmittel in der vorerwähnten Bestimmung jedes immunologisch verträgliche Markierungsmittel sein, welches eine quantitative Bestimmung zulässt, wie z.B. ein Radioisotop, ein Enzym, ein Fluoreszens oder ein Chemilumineszens oder dergleichen. Enzym- und Radioisotop-Markierungsmittel sind bevorzugt, wobei die letzteren besonders bevorzugt sind.

Unter den Radioisotopen, welche herkömmlicherweise bei Radioimmunverfahren verwendet werden, sind die Isotope von Jod bevorzugt, wobei Jod-125 besonders bevorzugt ist.

Das Insolubilisierungsmittel, welches verwendet wird, um eine feste Phase enthaltend Antigen-gebundenes CEA und eine flüssige Phase enthaltend ungebundenes CEA zu formen, kann jedes Material sein, welches für diesen Zweck bekannt ist. So z.B. können gewisse aliphatische Alkohole, Ionenaustauschharze und anorganische Salze sowie auch Antikörper, welche gegen den CEA-Antikörper gerichtet sind, die Bildung eines Proteinniederschlages bewirken, welcher Antigen-gebundenes CEA enthält. Ein bevorzugtes Insolubilisierungsmittel ist ein zweiter Antikörper, d.h. ein Antikörper, der gegen den CEA-Antikörper gerichtet ist, in immobilisierter Form, d.h. in insolubilisierter Form.

Bezüglich der vorerwähnten Immobilisierung des zweiten Antikörpers kann jede bekannte Technik zum Insolubilisieren einer immunologischen Komponente, wie z.B. Partikel verschiedener Grösse, Kügelchen, Stäbchen oder Streifen eines Trägermaterials, verwendet werden. Sofern als Insolubilisierungsmaterial ein Polymer verwendet wird, z.B. ein Styrolpolymer oder Copolymer, kann - entsprechend den individuellen Eigenschaften des Polymers - mehr als eine der oben erwähnten Formen verwendet werden. Ein besonders bevorzugtes Material zur Immobilisierung des zweiten Antikörpers ist ungesintertes Poly(vinylidenfluorid) welche z.B. in Form von feinen Partikeln oder als Film verwendet werden kann. Der Antikörper kann physikalisch an das immobilisierende Material gebunden sein oder nach konventionellen Methoden chemisch daran gebunden sein.

Die besonderen Verhältnisse, Inkubationszeiten und Temperaturen, welche für eine gegebene CEA-Bestimmung zu berücksichtigen sind, liegen im Hinblick auf die zahlreichen Publikationen bezüglich CEA im Bereich des Fachwissens. Bezüglich der Bestimmung von CEA in einem Radioimmunverfahren unter Verwendung der neuen Vorbehandlungsmethode gemäss vorliegender Erfindung wurde gefunden, dass es im Vergleich zu kommerziellen CEA-Bestimmungen möglich ist, ohne entsprechenden Verlust in der Empfindlichkeit, signifikant kleinere Volumen von Reagenzien und Proben zu verwenden. Dies ermöglicht die Verwendung kleinerer Reaktionsröhrchen, Ableseeinheiten und dergleichen. Bei Verwendung der Probenvorbehandlung gemäss vorliegender Erfindung besteht ein weiterer Vorteil darin, dass ungefähr eine fünffache Reduktion des Anteils an CEA-Antikörper vorgenommen werden kann. Dies bedeutet im Vergleich zu kommerziellen CEA-Radioimmunverfahren, dass Antiseren zu CEA weniger oft standardisiert werden müssen, was wesentliche Einsparungen hinsichtlich Zeit und Aufwand bedeutet.

Die CEA-Bestimmung nach Vorbehandlung der Probe gemäss vorliegender Erfindung zeigt einen weiteren Vorteil gegenüber den derzeitigen kommerziellen CEA-Radioimmunverfahren, indem die letzteren nur bis ungefähr 20 ng/ml CEA exakt sind. Im Fall der Chemotherapieüberwachung und im Falle der Bestimmung höherer Anteile an CEA ist es notwendig, einen unterschiedlichen Typ von Bestimmung anzuwenden, d.h. eine direkte Bestimmung zur Ueberwachung solch höherer Anteile. Dieser letztere Typ von Bestimmungsmethode kann für exakte CEA-Konzentrationen, welche bei einem Minimum von ungefähr 40 ng/ml beginnen, angewendet werden, was bedeutet, dass zwischen diesem Typ von Bestimmungsmethode und dem kommerziellen Radioimmunverfahren für CEA eine Lücke von ungefähr 20 ng/ml offen bleibt. Die Bestimmung von CEA nach Vorbehandlung der Probe gemäss vorliegender Erfindung erlaubt eine exakte Ablesung von CEA bis zu Konzentrationen von ungefähr 100 ng/ml, was bedeutet, dass die vorerwähnte Lücke gefüllt wird. Im weitern bedeutet es eine wesentliche Einsparung bezüglich Zeit und Geld für den Patienten, da keine Notwendigkeit besteht, eine zweite Bestimmung durchzuführen um CEA-Konzentrationen zu ermitteln, welche wesentlich über 20 ng/ml liegen.

Im Rahmen der vorliegenden Erfindung wurde gefunden, dass lediglich 0,1 ml der zu untersuchenden Probe bei der Durchführung der Bestimmung verwendet werden muss und dass ungefähr 5 µl CEA-Antiserum zur Probe und zum Standard gegeben werden müssen. Bevorzugte Reaktionszeiten für die Reaktion mit dem Antiserum und der nachfolgenden Reaktion mit dem markierten CEA sind von ungefähr 80 Minuten bis ungefähr 2 Stunden, wobei 90 Minuten besonders bevorzugt sind. Diese Inkubationen werden bei einer Temperatur von ungefähr 45°C durchgeführt. Bei Verwendung eines Enzyms als Markierungsmittel muss unter Umständen die Inkubationszeit und die besondere Temperatur angepasst werden, um eine Inaktivierung des Enzyms zu vermeiden, sofern das Enzym bei den oben angegebenen Temperaturen labil ist. Bei Verwendung eines immobilisierten zweiten Antikörpers als Insolubilisierungsmittel werden die Proben vorzugsweise bei Raumtemperatur während ungefähr 15-30 Minuten, vorzugsweise während ungefähr 20 Minuten, inkubiert. Die Bestimmung von CEA in der Probe wird nach herkömmlicher Art und Weise durch Vergleich mit einer Standardkurve durchgeführt. Das Ablesen der Konzentration an Markierungsmittel wird abhängig vom Typ des Markierungsmittels ebenfalls nach herkömmlichen Methoden durchgeführt, z.B. unter Verwendung eines Gamma-Szintillations-Spectrometers, wenn ein Radionuklid-Markierungsmittel verwendet wird.

Die nachfolgenden Beispiele illustrieren die Erfindung weiterhin. Sofern nicht anders angegeben, sind alle Temperaturen in Celsiusgraden angegeben.

## Beispiel 1

Die kommerzielle Bestimmung für CEA wird wie folgt durchgeführt:

Plasmaproben (0,5 ml; im Doppel) von Patienten mit Verdacht auf Colonkarzinom werden mit 2,5 ml kalter 1,2 molarer Perchlorsäure durch Mischen in einem Mischer vom Vortextyp während 30 Sekunden extrahiert und dann während 20 Minuten bei 1000 x g zentrifugiert. Die Ueberstände werden gesammelt und in einen Dialyseschlauch gegeben und dann gegen deionisiertes Wasser dialysiert, wobei das Wasser dreimal gewechselt wird und zwischen jedem Wechsel mindestens 3 Stunden gewartet wird. Es wird eine Schlussdialyse unter Verwendung eines Ammoniumacetatpuffers vom pH ungefähr 6,5 durchgeführt. Nach der Dialyse wird der Inhalt der Dialyseschläuche in Teströhrchen gegeben, worauf unter Mischen mit einem Vortexmischer 25 µl von kommerziell erhältlichem CEA-Antiserum zugegeben werden. Die Röhrchen werden während 30 Minuten bei 45° inkubiert. Zu jedem Röhrchen wird unter Mischen 25 µl $^{125}$J-CEA zugegeben, worauf die Röhrchen wiederum während 30 Minuten bei 45° inkubiert werden. Es werden 2,5 ml Zirconylphosphatgel (pH 6,25) zu jedem Röhrchen gegeben, worauf man während 5 Minuten bei 1000 x g zentrifugiert. Nach Entfernen der Ueberstände wird unter Mischen zu jedem Röhrchen 5 ml Ammoniumacetatpuffer vom pH 6,25 gegeben. Die Röhrchen werden erneut wie oben beschrieben zentrifugiert, und die Ueberstände werden entfernt. Der Anteil von gebundenem $^{125}$J-CEA im verbleibenden Gel wird während 1 Minute durch Zählen in einem Gamma-Szintillations-Spectrometer bestimmt.

Eine Standardinhibitionskurve wird wie folgt hergestellt:

Zu Paaren von Teströhrchen wird 5,0 ml einer 1:10-Verdünnung von EDTA-Puffer (pH 6,5) mit deionisiertem Wasser gegeben. In jedes Röhrchen wird eine CEA-Standard-basis, d.h. 0, 10, 25, 50 und 100 µl entsprechend 0, 1,25, 3,125, 6,25 und 12,5 ng/ml CEA-Aktivität gegeben, und die Röhrcheninhalte werden mit einem Mischer vom Vortextyp gemischt. Die Röhrchen werden dann wie oben angegeben mit Antiserum zu CEA und $^{125}$J-CEA behandelt. Es wird abgelesen und eine Kurve erstellt, welche zum Vergleich der Resultate dient, die mit den Patientenplasmen erhalten wurde.

## Beispiel 2

Eine Bestimmung von CEA unter Verwendung der Vorbehandlung der Probe gemäss vorliegender Erfindung wird wie folgt durchgeführt:

Plasmaproben (0,1 ml) werden mit 1,5 ml deionisiertem Wasser verdünnt und mit einem Mischer vom Vortextyp gemischt. Die Röhrchen werden während 10 Minuten in ein Wasserbad von 95° gestellt, wonach man sie aus dem Wasserbad entfernt und auf Raumtemperatur abkühlen lässt. Unter Rühren mit einem Vortexmischer werden zu jedem Röhrchen 5 µl kommerzielles CEA-Antiserum zugegeben. Die Röhrchen werden während 90 Minuten bei 45° inkubiert. Unter Mischen wird zu jedem Röhrchen 25 µl $^{125}$J-CEA gegeben, worauf die Röhrchen wiederum während 90 Minuten bei 45° inkubiert werden. Zu jedem Röhrchen wird 500 µl einer wässrigen Suspension eines Antikörpers gegen das CEA-Antiserum gegeben, welcher durch Adsorption an Partikel von Poly(vinylidenfluorid) insolubilisiert wurde. Die Röhrchen werden erneut gemischt und während 20 Minuten bei Raumtemperatur inkubiert. Die Proben werden dann während 10 Minuten bei 1000 x g zentrifugiert. Die Ueberstände werden dekantiert, worauf die Radioaktivität der Röhrchen in einem Gamma-Szintillations-Spectrometer bestimmt wird.

Eine Standardinhibitionskurve wird wie folgt hergestellt:

Zu Paaren von Teströhrchen werden 0,1 ml rekonstituiertes, lyophilisiertes, humanes Plasma gegeben, welches mit 1,5 ml deionisiertem Wasser verdünnt wurde, Der Inhalt der Röhrchen wird mit einem Vortextypmischer gerührt und dann während 10 Minuten bei 95° inkubiert. Zu jedem Paar von Röhrchen wird eine CEA-Standarddosis, d.h. 0, 5, 10, 25, 50 und 100 µl entsprechend 0, 0,625, 1,25, 3,125, 6,25 und 12,5 ng/ml CEA-Aktivität gegeben, worauf man mit einem Mischer vom Vortextyp mischt. Die Röhrchen werden wie oben angegeben mit Antiserum zu CEA, [125]J-CEA und insolubilisiertem zweiten Antikörper behandelt. Nach Ablesen wird eine Kurve erstellt, welche zum Vergleich der Resultate dient, welche mit den Plasmaproben der Patienten erhalten wurden.

In Tabelle I werden die Resultate für die Proben, welche nach der herkömmlichen Dialysemethode gemäss Beispiel 1 erhalten wurden, mit denjenigen Resultaten, die mit der erfindungsgemässen Methode gemäss Beispiel 2 erhalten wurden, verglichen.

## Tabelle I

| Probe-Nr. | Beispiel 1 | Beispiel 2 |
|---|---|---|
| 1 | 0,8 | 1,8 |
| 2 | 0,9 | 1,0 |
| 3 | 4,1 | 4,4 |
| 4 | 3,7 | 5,6 |
| 5 | 10,5 | 11,4 |
| 6 | 1,5 | 1,4 |
| 7 | 1,0 | 0,0 |
| 8 | 3,3 | 3,4 |
| 9 | 7,9 | 10,1 |
| 10 | 2,0 | 3,8 |
| 11 | 0,5 | 1,9 |
| 12 | 4,8 | 4,4 |
| 13 | 6,9 | 5,3 |
| 14 | 1,5 | 0,4 |
| 15 | 1,4 | 1,9 |
| 16 | 3,2 | 4,3 |
| 17 | 10,5 | 11,2 |
| 18 | 1,9 | 1,2 |
| 19 | 4,8 | 4,7 |
| 20 | 3,2 | 3,6 |
| 21 | 11,6 | 11,9 |

- 13 -

0033960

Die Resultate in Tabelle I zeigen klar, dass in Konzentrationen bis zu 20 ng/ml die Methoden von Beispiel 1 und 2 gut miteinander korrelieren (Korrelationskoeffizient entspricht 0,963; Steigung (slope) entspricht 1,03).

## Beispiel 3

Bei Proben die nach dem Verfahren von Beispiel 1 bestimmt wurden und bei denen das Resultat anzeigte, dass die CEA-Konzentration in der Probe oberhalb 20 ng/ml liegt, wurde das kommerzielle Verfahren zur Bestimmung solcher Konzentrationen wie folgt durchgeführt:

Plasmaproben (50 µl) werden zu Teströhrchen enthaltend 5 ml Ammoniumacetatpuffer (0,01M Acetat) vom pH 6,8 gegeben. Zu jedem Röhrchen wird 25 µl CEA-Antiserum gegeben, worauf man mit einem Mischer vom Vortextyp mischt. Die Röhrchen werden bei 45° während 30 Minuten inkubiert. Zu jedem Röhrchen werden dann 25 µl $^{125}$J-CEA gegeben, worauf die Röhrchen wiederum während 30 Minuten bei 45° inkubiert werden. 2,5 ml Zirconylphosphatgel vom pH 6,25 wird zu jedem Röhrchen gegeben, worauf die Röhrchen während 5 Minuten bei 1000 x g zentrifugiert werden. Nach Entfernen der Ueberstände gibt man unter Mischen 5 ml Ammoniumacetatpuffer vom pH 6,25 zu. Die Röhrchen werden wie vorher beschrieben erneut zentrifugiert und die Ueberstände entfernt. Der Anteil an gebundenem $^{125}$J-CEA im verbleibenden Gel wird durch Zählen in einem Gamma-Szintillations-Spectrometer während 1 Minute bestimmt.

Es wird wie folgt eine Standardinhibitionskurve hergestellt:

Zu je einem Paar von Teströhrchen wird 5,0 ml eines 0,01M Acetatpuffers vom pH 6,8 gegeben. Zu jedem Röhrchen wird 50 µl normales humanes Plasma gegeben, worauf man gründlich mischt. Zu jedem Paar von Röhrchen wird eine CEA-Standarddosis, d.h. 0, 10, 25, 50 und 100 µl entsprechend 0, 1,25, 3,125, 6,25 und 12,5 ng/ml CEA-Aktivität gegeben, worauf man mit einem Mischer vom Vortextyp mischt. Die Röhrchen werden dann wie oben beschrieben mit Antiserum zu CEA und $^{125}$J-CEA behandelt. Nach Ablesen wird eine Kurve erstellt, welche zum Vergleich der Resultate dient, die mit den Patientenplasmen erhalten wurden.

Dieselben Proben werden entsprechend dem Verfahren gemäss der vorliegenden Erfindung, d.h. gemäss Beispiel 2 analysiert. Ein Vergleich der erhaltenen Resultate wird in Tabelle II gegeben.

Tabelle II

| Probe-Nr. | Beispiel 2 | Beispiel 3 |
|-----------|-----------|-----------|
| 1 | 18,5 | 30,7 |
| 2 | 49,1 | 95,4 |
| 3 | 32,0 | 67,5 |
| 4 | 25,0 | 54,1 |
| 5 | 25,3 | 72,1 |
| 6 | 23,8 | 56,9 |
| 7 | 32,8 | 63,9 |
| 8 | 30,4 | 49,8 |
| 9 | 37,9 | 85,2 |
| 10 | 23,3 | 43,6 |
| 11 | 26,5 | 41,7 |
| 12 | 47,5 | 51,5 |
| 13 | 34,7 | 47,1 |
| 14 | 17,6 | 40,6 |
| 15 | 29,1 | 68,5 |
| 16 | 23,5 | 36,1 |
| 17 | 25,7 | 41,4 |
| 18 | 19,9 | 26,3 |
| 19 | 86,4 | 67,5 |
| 20 | 33,1 | 68,2 |
| 21 | 27,6 | 67,1 |
| 22 | 30,2 | 59,6 |
| 23 | 26,1 | 72,5 |
| 24 | 29,8 | 59,0 |

| | | |
|---|---|---|
| 25 | 28,2 | 55,5 |
| 26 | 17,8 | 36,7 |
| 27 | 20,6 | 38,6 |
| 28 | 91,5 | 91,8 |

Die in Tabelle II erhaltenen Resultate zeigen, dass man nach der Vorbehandlung der Proben gemäss der vorliegenden Erfindung Konzentrationen von CEA oberhalb von 20 ng/ml exakt messen kann. Durchgeführte Teste, bei denen die in diesem Beispiel bestimmten Proben auf eine Konzentration von unterhalb 20 ng/ml CEA verdünnt wurden und Bestimmen der CEA-Konzentration dieser verdünnten Proben nach der Methode von Beispiel 1 haben gezeigt, dass die Methode gemäss vorliegender Erfindung eine exaktere Bestimmung erhöhter CEA-Konzentrationen erlaubt, als das kommerziell erhältliche Verfahren.

## Patentansprüche

1. Verfahren zur Vorbehandlung einer menschlichen Serum- oder Plasma-Probe, welche zur Bestimmung der Konzentration von carcinoembryonischem Antigen verwendet werden soll, dadurch gekennzeichnet, dass man der Probe genügend Wasser zugibt um sie zu einer Verdünnung von ungefähr 1:8 zu ungefähr 1:50 zu bringen, Wärmen der verdünnten Probe während ungefähr 3-30 Minuten bei einer Temperatur, welche unterhalb derjenigen liegt, bei welcher die Proteine in der Probe koagulieren würden, wobei die Materialien in der Probe, welche mit der erwähnten Bestimmungsmethode interferieren würden, neutralisiert werden.

2. Verfahren nach Anspruch 1, dadurch gekennzeichnet, dass die Probe auf eine Verdünnung von ungefähr 1:16 gebracht wird.

3. Verfahren nach Anspruch 1 oder 2, dadurch gekennzeichnet, dass die verdünnte Probe während einer Zeit von ungefähr 5 bis ungefähr 10 Minuten erwärmt wird.

4. Verfahren nach einem der Ansprüche 1-3, dadurch gekennzeichnet, dass die verdünnte Probe auf eine Temperatur zwischen ungefähr 85 und ungefähr 105°C erwärmt wird.

5. Verfahren nach Anspruch 4, dadurch gekennzeichnet, dass die Probe auf eine Temperatur von ungefähr 95°C erwärmt wird.

6. Verfahren nach einem der Ansprüche 1-5, dadurch gekennzeichnet, dass das Wasser, welches zum Verdünnen der Probe verwendet wird, eine genügende Menge eines geeigneten Puffers enthält, um die Probe auf einen pH von ungefähr 7,5 bis ungefähr 9 abzupuffern.

\*\*\*